# EUROPÄISCHE PATENTSCHRIFT

(11) **EP 1 478 614 B1**
(45) Veröffentlichungstag und Bekanntmachung des Hinweises auf die Patenterteilung: **21.02.2007**
(21) Anmeldenummer: 03706423.5
(22) Anmeldetag: 04.02.2003
(51) Int. Cl.: C07C 51/265, C07C 51/31, C07C 63/16

(54) **VERFAHREN ZUR HERSTELLUNG VON PHTHALSÄUREANHYDRID**
METHOD FOR PRODUCING PHTHALIC ANHYDRIDE
PROCEDE DE PREPARATION D'ANHYDRIDE D'ACIDE PHTALIQUE

(30) Priorität: 19.02.2002 DE 10206989
(43) Veröffentlichungstag der Anmeldung: 24.11.2004
(73) Patentinhaber: BASF Aktiengesellschaft, 67056 Ludwigshafen (DE)
(72) Erfinder: STORCK, Sebastian, 68167 Mannheim (DE); ROSOWSKI, Frank, 68165 Mannheim (DE); BAIER, Michael, 68161 Mannheim (DE); TENTEN, Andreas, B-3090 Overijse (BE); WOLF, Hans-Josef, 67133 Maxdorf (DE)
(86) Internationale Anmeldenummer: PCT/EP2003/001066
(87) Internationale Veröffentlichungsnummer: WO 2003/070680

(56) Entgegenhaltungen:
- EP-A- 1 063 222
- WO-A-02/16299
- US-A- 4 284 571

## Beschreibung

Die vorliegende Erfindung betrifft ein Verfahren zur Herstellung von Phthalsäureanhydrid durch Gasphasenoxidation von Xylol, Naphthalin oder Gemischen davon in einem mit einem Wärmeträgermedium thermostatisierten Rohrbündelreaktor an drei oder mehr als drei verschiedenen, schichtweise angeordneten Festbettkatalysatoren.

Bekanntlich wird Phthalsäureanhydrid technisch durch katalytische Gasphasenoxidation von o-Xylol oder Naphthalin in Rohrbündelreaktoren hergestellt. Ausgangsmaterial ist ein Gemisch aus einem molekularen Sauerstoff enthaltenden Gas, beispielsweise Luft, und dem zu oxidierenden o-Xylol und/oder Naphthalin. Das Gemisch wird durch eine Vielzahl in einem Reaktor angeordneter Rohre (Rohrbündelreaktor) geleitet, in denen sich eine Schüttung mindestens eines Katalysators befindet. Zur Temperaturregelung sind die Rohre von einem Wärmeträgermedium, beispielsweise einer Salzschmelze umgeben. Trotzdem kann es in der Katalysatorschüttung zur Ausbildung lokaler Temperaturmaxima (Hot spots) kommen, in denen eine höhere Temperatur herrscht als im übrigen Teil der Katalysatorschüttung. Diese Hot spots geben Anlaß zu Nebenreaktionen, wie der Totalverbrennung des Ausgangsmaterials, oder führen zur Bildung unerwünschter, vom Reaktionsprodukt nicht oder nur mit viel Aufwand abtrennbarer Nebenprodukte. Außerdem kann der Katalysator ab einer bestimmten Hot spot-Temperatur irreversibel geschädigt werden.

Die Hot spot-Temperaturen liegen üblicherweise im Temperaturbereich von 400 bis 500°C, insbesondere im Temperaturbereich von 410 bis 460°C. Hot spot-Temperaturen über 500°C führen zu einer starken Abnahme der erreichbaren PSA-Ausbeute sowie der Katalysatorstandzeit. Zu niedrige Hot spot-Temperaturen führen dagegen zu einem zu großen Gehalt an Unteroxidationsprodukten im Phthalsäureanhydrid (insbesondere Phthalid), wodurch die Produktqualität entscheidend beeinträchtigt wird. Die Hot spot-Temperatur hängt von der Xylolbeladung des Luftstroms, von der Belastung des Katalysators mit dem Xylol/Luft-Gemisch, vom Alterungszustand des Katalysators, von den für den Festbettreaktor charakteristischen Wärmeübergangsverhältnissen (Reaktorrohr, Salzbad) und von der Salzbadtemperatur ab.

Zur Abschwächung dieser Hot spots wurden verschiedene Maßnahmen getroffen, die unter anderem in der DE 25 46 268 A, EP 286 448 A, DE 29 48 163 A, EP 163 231 A, DE 41 09 387 A, WO 98/37967 und DE 198 23 362 A aufgeführt sind. Insbesondere wurde, wie in der DE 40 13 051 A beschrieben, dazu übergegangen, unterschiedlich aktive Katalysatoren schichtweise in der Katalysatorschüttung anzuordnen, wobei in der Regel der weniger aktive Katalysator zum Gaseintritt hin und der aktivere Katalysator zum Gasaustritt hin gelegen ist. Das Verfahren wird so durchgeführt, dass ein zweistufiges Salzbad verwendet wird, wobei die Salzbadtemperatur der ersten Reaktionszone in Strömungsrichtung des Reaktionsgemisches um 2 bis 20°C höher gehalten wird als die Salzbadtemperatur der zweiten Reaktionszone. Das Katalysatorvolumen der ersten Reaktionszone beträgt dabei 30 bis 75 Vol.-% und das der zweiten Reaktionszone 25 bis 70 Vol.-%. Die Temperatur des Hot spots in der ersten Reaktionszone liegt höher als die in der zweiten Reaktionszone. Die Differenz der Hot spot-Temperaturen bei den in den Beispielen beschriebenen Fahrweisen liegt erheblich niedriger als 50°C.

Die DE 28 30 765 A beschreibt einen Rohrbündelrektor, der auch zur Herstellung von Phthalsäureanhydrid mit einem in zwei Reaktionszonen befindlichen Katalysator geeignet ist. Die Reaktionstemperatur in der vom Gaseintritt her gesehen zweiten Reaktionszone ist dabei höher als die in der ersten Reaktionszone.

Die DE 29 48 163 A beschreibt ein Verfahren zur Herstellung von Phthalsäureanhydrid mit zwei schichtweise angeordneten unterschiedlichen Katalysatoren, wobei der Katalysator der ersten Schicht 30 bis 70% und der Katalysator der zweiten Schicht 70 bis 30% der Gesamtlänge der Katalysatorschicht ausmacht. Damit soll die Temperatur der Hot spots erniedrigt werden. Es hat sich jedoch gezeigt, dass die Phthalsäureanhydridausbeute selbst bei den in dieser Publikation verwendeten geringen Beladungen an o-Xylol im Ausgangsgasgemisch (maximal 85 g/Nm³) zu wünschen übrig läßt. Einen ähnlichen Offenbarungsgehalt besitzt die DE 30 45 624 A.

Die DE 198 23 262 beschreibt ein Verfahren zur Herstellung von Phthalsäureanhydrid mit mindestens drei in Schichten übereinander angeordneten Schalenkatalysatoren, wobei die Katalysatoraktivität von Schicht zu Schicht von der Gaseintrittsseite zur Gasaustrittsseite ansteigt. Der Unterschied in der Hot spot-Temperatur von Katalysator zu Katalysator liegt bei diesem Verfahren bei maximal 10°C.

Die EP-A 1 063 222 beschreibt ein Verfahren zur Herstellung von Phthalsäureanhydrid, welches in einem oder mehreren Festbettreaktoren durchgeführt wird. Die Katalysatorbetten in den Reaktoren weisen drei oder mehr als drei individuelle, im Reaktor aufeinander folgende Katalysator-Schichten auf. Nach Durchgang durch die erste Katalysatorschicht unter den Reaktionsbedingungen sind 30 bis 70 Gew.-% des eingesetzten o-Xylol, Naphthalins oder des Gemisch aus beiden umgesetzt. Nach der zweiten Schicht sind 70 Gew.-% oder mehr umgesetzt.

Jedoch können die gemäß der EP-A 1 063 222 erhaltenen Ergebnisse noch nicht befriedigen, weil die Reaktionswärme und der Umsatz der Ausgangsstoffe nicht gleichmäßig über den Reaktor, insbesondere die Katalysatorschüttung verteilt sind, wie aus dem Hot-Spot-Profil in der dortigen Figur 5 zu erkennen ist. Somit tritt eine unterschiedliche Alterung der Katalysatorschichten ein, welche wiederum nach längerer Betriebsdauer eine Abnahme der Ausbeute zur Folge hat.

Der vorliegenden Erfindung lag daher die Aufgabe zugrunde, ein Verfahren zur Herstellung von Phthalsäureanhydrid zur Verfügung zu stellen, das Phthalsäureanhydrid auch bei hohen o-Xylol- oder Naphthalinbeladungen und bei hohen Raumgeschwindigkeiten mit hoher Ausbeute ergibt, wobei die Reaktionswärme gleichmäßiger über die Länge der gesamten Katalysatorschüttung verteilt ist und zu einer verlängerten Lebensdauer der Katalysatoren beiträgt.

Überraschenderweise wurde nun gefunden, dass diese Aufgabe gelöst wird, wenn man die Phthalsäureanhydridherstellung an drei oder mehr, vorzugsweise drei bis fünf, schichtweise angeordneten, unterschiedlich aktiven ringförmigen Träger-Katalysatoren durchführt, wobei man die Umsetzung so steuert, dass die Maximaltemperatur in der, vom Gaseintritt (in Strömungsrichtung) her gesehen, zweiten Katalysatorschicht um 10 bis 40°C niedriger ist als diejenige in der ersten Katalysatorschicht, die Maximaltemperatur in der vom Gaseintritt (in Strömungsrichtung) her gesehen dritten Katalysatorschicht um 30 bis 100°C niedriger ist als diejenige in der ersten Katalysatorschicht und die Maximaltemperatur in der ersten Katalysatorschicht weniger als 470°C beträgt.

Gegenstand der vorliegenden Erfindung ist daher ein Verfahren zur Herstellung von Phthalsäureanhydrid durch Gasphasenoxidation von Xylol, Naphthalin oder Gemischen davon in einem mit einem wärmeträgermedium thermostatisierten Rohrbündelreaktor an drei oder mehr als drei verschiedenen, schichtweise angeordneten ringförmigen Festbett-Träger-Katalysatoren, wobei das Verfahren so durchgeführt wird, dass die Maximaltemperatur in der in Strömungsrichtung zweiten Katalysatorschicht 10 bis 40°C niedriger ist als die Maximaltemperatur in der ersten Katalysatorschicht, die Maximaltemperatur in der vom Gaseintritt her gesehenen dritten Schicht um 30 bis 100°C niedriger ist als diejenige der ersten Katalysatorschicht und die Maximaltemperatur in der ersten Katalysatorschicht weniger als 470°C beträgt.

Vorzugsweise ist die Maximaltemperatur in der vom Gaseintritt (in Strömungsrichtung) her gesehenen dritten Katalysatorschicht 40 bis 80°C niedriger als die Maximaltemperatur in der ersten Katalysatorschicht.

Weiter wird das Verfahren so durchgeführt, dass die Maximaltemperatur in der ersten Katalysatorschicht vorzugsweise weniger als 450°C beträgt.

Die Differenz in den Maximaltemperaturen kann auf unterschiedliche Weise eingestellt werden. Beispielsweise kann dies durch Erhöhung des Eingangsdruckes des Ausgangsgasgemisches um bis zu 10% oder durch Erniedrigung der zur Oxidation verwendeten Luftmenge um bis zu 20% erfolgen. Vorzugsweise aber erfolgt die Steuerung der Temperaturdifferenz durch das Schüttungslängenverhältnis der drei oder mehr Katalysatorschichten oder durch die Temperatur des Wärmeträgermediums (im Folgenden wird stets auf das bevorzugte Wärmeträgermedium, nämlich ein Salzbad, Bezug genommen), insbesondere wenn die drei oder mehr Katalysatorschichten durch unterschiedliche Salzbadkreisläufe thermostatisiert werden. Die Schüttungslänge der ersten Katalysatorschicht macht vorzugsweise mehr als 30%, insbesondere mehr als 40% der Länge der gesamten Katalysatorschüttung aus.

Wird die Salzbadtemperatur zur Steuerung eingesetzt, so führt eine Erhöhung der Salzbadtemperatur zu einer Erhöhung der Maximaltemperatur in der ersten Katalysatorschicht und zu einer Erniedrigung in der zweiten und jeder folgenden Katalysatorschicht. Im Allgemeinen genügt daher eine geringfügige Erhöhung oder Erniedrigung, z.B. um 1, 2 oder 3°C, um die gewünschte Differenz der Maximaltemperaturen einzustellen. Wenn die drei oder mehr Katalysatorschichten durch unterschiedliche Salzbadkreisläufe temperiert werden, wird der obere Salzbadkreislauf, d.h. der die erste Katalysatorschicht temperierende Salzbadkreislauf, vorzugsweise um 0,5 bis 5°C höher betrieben als der untere Salzbadkreislauf. Alternativ wird die Temperatur des die zweite Katalysatorschicht temperierenden Salzbads um bis zu 10°C abgesenkt und die Temperatur des die dritte Katalysatorschicht temperierenden Salzbades um weitere 10°C abgesenkt.

Die Standzeit des Katalysators beträgt im Allgemeinen etwa 4 bis 5 Jahre. Im Laufe der Zeit läßt die Aktivität des Katalysators im Allgemeinen etwas nach. Dadurch kann die Differenz der Maximaltemperaturen zwischen erster und dritter Katalysatorschicht unter den Mindestwert von 30°C fallen. Sie läßt sich dann durch die oben beschriebene Erhöhung der Salzbadtemperatur wieder auf einen Wert von 30°C oder darüber einstellen. Vorzugsweise führt man das Verfahren so durch, dass die Differenzen der Maximaltemperaturen mindestens während der ersten 50%, insbesondere mindestens während der ersten 70%, besonders bevorzugt mindestens während der ersten 90% der Katalysatorstandzeit und mit besonderem Vorteil im Wesentlichen während der gesamten Katalysatorstandzeit erhalten bleiben.

Die Bestimmung der Maximaltemperatur bzw. der Hot spot-Temperatur erfolgt in bekannter Weise, z.B. durch Einbau einer Vielzahl von Thermoelementen in den Reaktor.

Als Katalysatoren sind oxidische Trägerkatalysatoren geeignet. Zur Herstellung von Phthalsäureanhydrid durch Gasphasenoxidation von o-Xylol oder Naphthalin verwendet man ringförmige Träger aus einem Silikat, Siliciumcarbid, Porzellan, Aluminiumoxid, Magnesiumoxid, Zinndioxid, Rutil, Aluminiumsilikat, Magnesiumsilicat (Steatit), Zirkoniumsilicat oder Cersilicat oder Mischungen davon. Als katalytisch aktiver Bestandteil dient im Allgemeinen neben Titandioxid, insbesondere in Form seiner Anatasmodifikation, Vanadiumpentoxid. Weiter können in der katalytisch aktiven Masse in geringen Mengen eine Vielzahl anderer oxydischer Verbindungen enthalten sein, die als Promotoren die Aktivität und Selektivität des Katalysators beeinflussen, beispielsweise indem sie seine Aktivität absenken oder erhöhen. Derartige Promotoren sind beispielsweise die Alkalimetalloxide, Thallium(I)oxid, Aluminiumoxid, Zirkoniumoxid, Eisenoxid, Nickeloxid, Cobaltoxid, Manganoxid, Zinnoxid, Silberoxid, Kupferoxid, Chromoxid, Molybdänoxid, Wolframoxid, Iridiumoxid, Tantaloxid, Nioboxid, Arsenoxid, Antimonoxid, Ceroxid und Phosphorpentoxid. Die Alkalimetalloxide wirken beispielsweise als die Aktivität vermindernde und die Selektivität erhöhende Promotoren, wohingegen oxidische Phosphorverbindungen, insbesondere Phosphorpentoxid, die Aktivität des Katalysators erhöhen, aber dessen Selektivität vermindern. Brauchbare Katalysatoren sind beispielsweise beschrieben in DE 25 10 994, DE 25 47 624, DE 29 14 683, DE 25 46 267, DE 40 13 051, WO 98/37965 und WO 98/37967. Besonders bewährt haben sich sogenannte Schalenkatalysatoren, bei denen die katalytisch aktive Masse schalenförmig auf den Träger aufgebracht ist (siehe z.B. DE 16 42 938 A, DE 17 69 998 A und WO 98/37967).

Der weniger aktive Katalysator wird so im Festbett angeordnet, dass das Reaktionsgas zuerst mit diesem Katalysator und erst im Anschluß daran mit dem aktiveren Katalysator in der zweiten Schicht in Kontakt kommt. Anschließend kommt das Reaktionsgas mit den noch aktiveren Katalysatorschichten in Kontakt. Die unterschiedlich aktiven Katalysatoren können auf die gleiche oder auf unterschiedliche Temperaturen thermostatisiert werden. Im Allgemeinen wird in der ersten, zum Gaseintritt hin gelegenen Katalysatorschicht ein mit Alkalimetalloxiden dotierter und in der zweiten Reaktionszone ein mit weniger Alkalimetalloxiden und/Phosphorverbindungen und/oder weiteren Promotoren dotierter Katalysator eingesetzt. In der dritten Katalysatorschicht wird ein mit noch weniger Alkalimetalloxiden bzw. Phosphorverbindungen und/oder weiteren Promotoren dotierter Katalysator eingesetzt.

Besonders bevorzugt sind Katalysatoren folgender Zusammensetzung:
- für die erste Schicht:
   3 bis 5 Gew.-% Vanadiumpentoxid
   0,1 bis 1 Gew.-% eines Alkalimetalloxids, z.B. Cäsiumoxid
   94 bis 96,9 Gew.-% Titandioxid
- für die zweite Schicht:
   4 bis 7 Gew.-% Vanadiumpentoxid
   0 bis 0,5 Gew.-% eines Alkalimetalloxids, z.B. Cäsiumoxid
   0,05 bis 0,4 Gew.-% Phosphorpentoxid (berechnet als P)
   dem Rest zu 100 Gew.-% Titandioxid
- für die dritte Schicht:
   6 bis 9 Gew.-% Vanadiumpentoxid
   0 bis 0,3 Gew.-% eines Alkalimetalloxids, z.B. Cäsiumoxid
   0,05 bis 0,4 Gew.-% Phosphorpentoxid (berechnet als P) gegebenenfalls 1 bis 5 Gew.-% eines weiteren Promotors, insbesondere Sb₂O₃
   85,3 bis 93,95 Gew.-% Titandioxid

Im Allgemeinen erfolgt die Umsetzung so, dass in der ersten Reaktionszone der größte Teil des im Reaktionsgas enthaltenen o-Xylols und/oder Naphthalins umgesetzt wird.

Die Katalysatoren werden zur Reaktion schichtweise in die Rohre eines Rohrbündelreaktors gefüllt. Über die so bereitete Katalysatorschüttung wird das Reaktionsgas bei Temperaturen von im Allgemeinen 300 bis 450°C, vorzugsweise 320 bis 420°C und besonders bevorzugt 340 bis 400°C, und bei einem Überdruck von im Allgemeinen 0,1 bis 2,5 bar, vorzugsweise 0,3 bis 1,5 bar, und mit einer Raumgeschwindigkeit von im Allgemeinen 750 bis 5000 h⁻¹, vorzugsweise 2000 bis 5000 h⁻¹, geleitet. Das dem Katalysator zugeführte Reaktionsgas (Ausgangsgasgemisch) wird im Allgemeinen durch Vermischen eines molekularen Sauerstoff enthaltenden Gases, das außer Sauerstoff noch geeignete Reaktionsmoderatoren und/oder Verdünnungsmittel, wie Dampf, Kohlendioxid und/oder Stickstoff enthalten kann, mit dem zu oxidierenden o-Xylol oder Naphthalin erzeugt. Das Reaktionsgas enthält im Allgemeinen 1 bis 100 mol%, vorzugsweise 2 bis 50 mol% und besonders bevorzugt 10 bis 30 mol% Sauerstoff. Im Allgemeinen wird das Reaktionsgas mit 5 bis 140 g/Nm³ Gas, vorzugsweise 60 bis 120 g/Nm³ und besonders bevorzugt 80 bis 120 g/Nm³ o-Xylol und/oder Naphthalin beladen.

Gewünschtenfalls kann man für die Phthalsäureanhydridherstellung noch einen nachgeschalteten Finishing-Reaktor vorsehen, wie er beispielsweise in der DE 198 07 018 oder DE 20 05 969 A beschrieben ist. Als Katalysator verwendet man dabei im Vergleich zum Katalysator der dritten Schicht vorzugsweise einen noch aktiveren Katalysator. Insbesondere weist dieser Katalysator folgende Zusammensetzung auf:
6 bis 9 Gew.-% Vanadiumpentoxid
1 bis 5 Gew.-% eines die Aktivität fördernden Promotors, insbesondere Sb₂O₃
0,1 bis 0,5 Gew.-% Phosphorpentoxid (berechnet als P)
85,5 bis 92,9 Gew.-% Titandioxid.

Das erfindungsgemäße Verfahren hat den Vorteil, dass sich Phthalsäureanhydrid auch bei hohen Beladungen mit o-Xylol und/oder Naphthalin und bei hohen Raumgeschwindigkeiten mit hoher Ausbeute und geringen Konzentrationen an Nebenprodukt, insbesondere Phthalid, herstellen läßt. Unter den Bedingungen des erfindungsgemäßen Verfahrens ist die Phthalid-Konzentration nicht höher als 0,1 Gew.-%, bezogen auf PSA. Die Vorteile des erfindungsgemäßen Verfahrens kommen besonders zum Tragen, wenn das eingesetzte Katalysatorsystem durch Alterung an Aktivität verliert. Auch nach langer Laufzeit kommt es nur zu einem unwesentlichen Anstieg der Maximaltemperatur in der zweiten Katalysatorschicht.

Die erfindungsgemäße Temperatursteuerung ist auch brauchbar bei der Herstellung anderer Produkte durch katalytische Gasphasenoxidation, wie z.B. Acrylsäure (aus Propen), Maleinsäureanhydrid (aus Benzol, Buten oder Butadien), Pyromellithsäureanhydrid (aus Durol), Benzoesäure (aus Toluol), Isophthalsäure (aus m-Xylol), Terephthalsäure (aus p-Xylol), Acrolein (aus Propen), Methacrylsäure (aus Isobuten), Naphthochinon (aus Naphthalin), Anthrachinon (aus Anthracen), Acrylnitril (aus Propen) und Methacrylnitril (aus Isobuten).

Die nachfolgenden Beispiele erläutern die Erfindung ohne sie zu begrenzen.

### Beispiele

### 1) Herstellung der Katalysatoren I - IV

### Katalysator I:

50 kg Steatit (Magnesiumsilikat)-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 28,6 kg Anatas mit einer BET-Oberfläche von 20 m²/g, 2,19 kg Vanadyloxalat, 0,176 kg Cäsiumsulfat, 44,1 kg Wasser und 9,14 kg Formamid besprüht, bis das Gewicht der aufgetragenen Schicht 10,5% des Gesamtgewichts des fertigen Katalysators betrug (nach Calcination bei 450°C).

Die auf diese Weise aufgebrachte katalytisch aktive Masse, also die Katalysatorschale, bestand aus 4,0 Gew.-% Vanadium (berechnet als V₂O₅), 0,4 Gew.-% Cäsium (berechnet als Cs) und 95,6 Gew.-% Titandioxid.

### Katalysator II:

Es wurde wie bei der Herstellung von Katalysator I gearbeitet, jedoch wurden 0,155 kg Cäsiumsulfat eingesetzt, was zu einem Gehalt von 0,35 Gew.-% Cäsium (berechnet als Cs) führte.

### Katalysator III

50 kg Steatit (Magnesiumsilikat)-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 28,6 kg Anatas mit einer BET-Oberfläche von 20 m²/g, 4,11 kg Vanadyloxalat, 1,03 kg Antimontrioxid, 0,179 kg Ammoniumdihydrogenphosphat, 0,045 kg Cäsiumsulfat, 44,1 kg Wasser und 9,14 kg Formamid besprüht, bis das Gewicht der aufgetragenen Schicht 10,5% des Gesamtgewichts des fertigen Katalysators betrug (nach Calcination bei 450°C).

Die auf diese Weise aufgebrachte katalytisch aktive Masse, also die Katalysatorschale, bestand aus 0,15 Gew.-% Phosphor (berechnet als P), 7,5 Gew.-% Vanadium (berechnet als V₂O₅), 3,2 Gew.-% Antimon (berechnet als Sb₂O₃), 0,1 Gew.-% Cäsium (berechnet als Cs) und 89,05 Gew.-% Titandioxid.

### Katalysator IV

50 kg Steatit (Magnesiumsilikat)-Ringe mit einem äußeren Durchmesser von 8 mm, einer Länge von 6 mm und einer Wandstärke von 1,5 mm wurden in einer Dragiertrommel auf 160°C erhitzt und mit einer Suspension aus 28,6 kg Anatas mit einer BET-Oberfläche von 11 m²/g, 3,84 kg Vanadyloxalat, 0,80 kg Antimontrioxid, 0,239 kg Ammoniumdihydrogenphosphat, 44,1 kg Wasser und 9,14 kg Formamid besprüht, bis das Gewicht der aufgetragenen Schicht 12,5% des Gesamtgewichts des fertigen Katalysators betrug (nach Calcination bei 450°C).

Die auf diese Weise aufgebrachte katalytisch aktive Masse, also die Katalysatorschale, bestand aus 0,2 Gew.-% Phosphor (berechnet als P), 7,0 Gew.-% Vanadium (berechnet als V₂O₅), 2,5 Gew.-% Antimon (berechnet als Sb₂O₃) und 90,3 Gew.-% Titandioxid.

### 2) Oxidation von o-Xylol

### 2a) Herstellung von PSA - erfindungsgemäß und Vergleich (Einstellung der Differenzen der Maximaltemperaturen durch Schüttungslängenvariation)

Von unten nach oben wurden in einem 10 1-Rohrreaktor (99 Normalrohre und 2 Thermorohre) jeweils Katalysator III (Vergleich: 1,30 m; erfindungsgemäß: 0,70 m), Katalysator II (erfindungsgemäß: 0,80 m) und anschließend Katalysator I (1,70 m (Vergleich); erfindungsgemäß 1,50 m) in jedes der 3,60 m langen Eisenrohre mit einer lichten Weite von 25 mm (Thermorohre 29 mm mit Thermohülse 10 mm lichte Weite und eingebauten 30-fach Thermoelementen (alle 10 cm)) eingefüllt. Mittels Druckabgleich wurde dafür gesorgt, daß an jedem Rohreingang der gleiche Eingangsdruck anlag. Gegebenenfalls wurde bei den 99 Normalrohren noch etwas Katalysator I hinzugefügt bzw. abgesaugt; bei den 2 Thermorohren wurde der Druckabgleich durch Zusatz von Inertmaterial in Form von Steatitkugeln bzw. Quarzkugeln erreicht. Die Eisenrohre waren zur Temperaturregelung von einer Salzschmelze umgeben, die sich in zwei getrennten Salzbädern befand. Das untere Salzbad umgab die Rohre vom unteren Rohrboden bis zu einer Höhe von 1,30 m, das obere Salzbad umgab die Rohre von der Höhe 1,30 m bis zum oberen Rohrboden. Durch die Rohre wurden stündlich von oben nach unten 4,0 Nm³ Luft pro Rohr mit Beladungen an 98,5 gew.-%igem o-Xylol von 100 g/Nm³ Luft (nach einer etwa zweimonatigen Hochfahrzeit) geleitet. Nach Verlassen des Hauptreaktors wurde der Rohproduktgasstrom auf 280 bis 290°C abgekühlt und noch durch einen adiabatischen Finishing-Reaktor (Innendurchmesser 0,45 m, Höhe 0,99 m), gefüllt mit 100 kg des Katalysators IV, geleitet.

Dabei wurden die in nachfolgender Tabelle aufgelisteten Daten erhalten (Fahrtag = Betriebstag ab dem ersten Start-up des Katalysators; SBT oben = Salzbadtemperatur des zum Reaktoreingang hin gelegenen Salzbades; SBT unten = Salzbadtemperatur des zum Reaktorausgang hin gelegenen Salzbades; Tₘₐₓ oben = Maximaltemperatur des zum Reaktoreingang hin gelegenen Katalysators; Tₘₐₓ unten = Maximaltemperatur des zum Reaktorausgang hin gelegenen Katalysators; PHD- bzw. Xylolgehalt = Phthalid- bzw. Xylolgehalt des Rohproduktgases nach dem Finishing-Reaktor bezüglich Phthalsäureanhydrid; PSA-Ausbeute = PSA-Ausbeute in Gew.-% bezüglich 100%igem Xylol aus der Analyse des Rohproduktgases nach dem Finishing-Reaktor.

| Schüttung | Fahrtag | SBT-oben SBT-unten | Tₘₐₓ-oben | Tₘₐₓ-mitte | Tₘₐₓ-unten | ΔTₘₐₓ oben-mitte | ΔTₘₐₓ oben-unten | PSA-Ausbeute |
|---|---|---|---|---|---|---|---|---|
| | [d] | [°C] | [°C] | [°C] | | [°C] | [°C] | [%] |
| | | | | | | | | |
| Vergleich | 100 | 348 / 348 | 434 | - | 366 | - | 68 | 113,1 |
| 170/130 | 150 | 348 / 348 | 434 | | 375 | | 57 | 112,9 |
| | 200 | 348 / 348 | 421 | | 390 | | 31 | 112,0 |
| | 250 | 348 / 348 | 419 | | 394 | | 25 | 111,3 |
| | | | | | | | | |
| Erfindungsgemäß 150/80/70 | 100 | 348 / 348 | 430 | 400 | 360 | 30 | 70 | 113,3 |
| | 150 | 348 / 348 | 431 | 402 | 359 | 29 | 71 | 113,1 |
| | 200 | 348 / 348 | 425 | 413 | 361 | 12 | 64 | 112,9 |
| | 250 | 348 / 348 | 421 | 411 | 362 | 10 | 59 | 112,7 |

### 2b) Herstellung von PSA - nicht erfindungsgemäß (Temperaturvariation und Temperaturstrukturierung)

Bei der in 2a) als Vergleichsversuch betriebenen Katalysatorkombination wurde nach 250 d Fahrzeit mittels Temperaturstrukturierung (SBT unten erniedrigt oder SBT oben erhöht) bzw. Temperaturvariation (SBT unten und oben erhöht) eine Temperaturdifferenz > 40°C eingestellt. Alle anderen Versuchsbedingungen wurden gegenüber Versuch 2a) nicht geändert.

Dabei wurden die in nachfolgender Tabelle aufgelisteten Daten erhalten (Fahrtag = Betriebstag ab dem ersten Start-up des Katalysators; SBT oben = Salzbadtemperatur des zum Reaktoreingang hin gelegenen Salzbades; SBT unten = Salzbadtemperatur des zum Reaktorausgang hin gelegenen Salzbades; Tₘₐₓ oben = Maximaltemperatur des zum Reaktoreingang hin gelegenen Katalysators; Tₘₐₓ unten = Maximaltemperatur des zum Reaktorausgang hin gelegenen Katalysators; PHD- bzw. Xylolgehalt = Phthalid- bzw. Xylolgehalt des Rohproduktgases nach dem Finishing-Reaktor bezüglich Phthalsäureanhydrid; PSA-Ausbeute = PSA-Ausbeute in Gew.-% bezüglich 100%igem Xylol aus der Analyse des Rohproduktgases nach dem Finishing-Reaktor.

| Schüttung 170/130 | Fahrtag | SBT-oben/ SBT-unten | Tₘₐₓ oben | Tₘₐₓ unten | Temperaturdifferenz | PSA-Ausbeute |
|---|---|---|---|---|---|---|
| | [d] | [°C] | [°C] | [°C] | [°C] | [m/m%] |
| | | | | | | |
| Vergleich ohne Temperatur-Strukturierung | 250 | 348/348 | 419 | 394 | 25 | 111,3 |
| | | | | | | |
| nicht erfindungsgemäß mit | 252 | 349/349 | 428 | 387 | 41 | 112,3 |
| Temperatur-Erhöhung | 254 | 350/350 | 437 | 381 | 56 | 112,5 |
| nicht erfindungsgemäß mit | 256 | 349/348 | 429 | 385 | 44 | 112,5 |
| Temperatur-Strukturierung | 258 | 350/348 | 438 | 379 | 58 | 112,8 |
| | 260 | 348/343 | 419 | 381 | 38 | 112,0 |
| | 262 | 348/338 | 418 | 370 | 48 | 112,9 |
| | 264 | 348/335 | 419 | 365 | 54 | 113,1 |

Die unter 2a) angegebenen Ergebnisse zeigen, dass die PSA-Ausbeute mit der Differenz der Maximaltemperatur korreliert, d.h. PSA wird unter praktisch relevanten Betriebsbedingungen mit hoher Ausbeute erhalten, wenn die Temperaturdifferenzen zwischen der ersten und zweiten Katalysatorschicht zwischen 10 und 40°C und zwischen der ersten und dritten Katalysatorschicht zwischen 30 und 100°C liegen.

Die unter 2b) angegebenen Ergebnisse zeigen, dass es bei zu niedriger Differenz der Maximaltemperatur genügt, entweder die Salzbadtemperatur oben und unten gleichzeitig und geringfügig zu erhöhen oder bei konstanter Temperatur des oberen Salzbades die Temperatur des unteren Salzbades abzusenken, um die Differenz der Maximaltemperaturen in einer zweifach strukturierten Schüttung zu erhöhen.

## Patentansprüche

1. Verfahren zur Herstellung von Phthalsäureanhydrid durch Gasphasenoxidation von Xylol, Naphthalin oder Gemischen davon in einem mit einem Wärmeträgermedium thermostatisierten Rohrbündelreaktor an drei oder mehr als drei verschiedenen, schichtweise angeordneten ringförmigen Festbett-Träger-Katalysatoren, wobei das Verfahren so durchgeführt wird, dass die Maximaltemperatur in der in Strömungsrichtung zweiten Katalysatorschicht um 10 bis 40°C niedriger ist als die Maximaltemperatur in der ersten Katalysatorschicht, die Maximaltemperatur in der vom Gaseintritt her gesehenen dritten Schicht um 30 bis 100°C niedriger ist als diejenige der ersten Katalysatorschicht und die Maximaltemperatur in der ersten Katalysatorschicht weniger als 470°C beträgt.

2. Verfahren nach Anspruch 1, **dadurch gekennzeichnet, dass** die Maximaltemperatur in der dritten Katalysatorschicht um 40 bis 80°C niedriger ist als in der ersten Katalysatorschicht.

3. Verfahren nach einem der vorhergehenden Ansprüche, **dadurch gekennzeichnet, dass** die Temperaturdifferenz zwischen der Maximaltemperatur in der ersten Katalysatorschicht und der Maximaltemperatur in der zweiten und dritten Katalysatorschicht über das Schüttungslängenverhältnis der Katalysatorschichten gesteuert wird.

4. Verfahren nach Anspruch 3, **dadurch gekennzeichnet, dass** die Schüttungslänge der ersten Katalysatorschicht mehr als 30% der Länge der gesamten Katalysatorschüttung beträgt.

5. Verfahren nach Anspruch 3, **dadurch gekennzeichnet, dass** die Schüttungslänge der ersten Katalysatorschicht mehr als 40% der Länge der gesamten Katalysatorschüttung beträgt.

6. Verfahren nach Anspruch 1, **dadurch gekennzeichnet, dass** die Temperaturdifferenz zwischen der Maximaltemperatur in der ersten und der Maximaltemperatur in der zweiten Katalysatorschicht über die Temperatur des Wärmeträgermediums gesteuert wird.

7. Verfahren nach einem der vorhergehenden Ansprüche, **dadurch gekennzeichnet, dass** die Maximaltemperatur in der ersten Katalysatorschicht weniger als 450°C beträgt.

8. Verfahren nach einem der vorhergehenden Ansprüche, **dadurch gekennzeichnet, dass** man eine Gasphase mit einer Beladung von 80 bis 140 g o-Xylol und/oder Naphthalin pro Nm³ Gasphase verwendet.

9. Verfahren nach einem der vorhergehenden Ansprüche, **dadurch gekennzeichnet, dass** die Temperatur des Wärmeträgers ≤ 360°C beträgt.

10. Verfahren nach einem der vorhergehenden Ansprüche, **dadurch gekennzeichnet, dass** die Raumgeschwindigkeit des Gasgemisches ≥ 2000 h⁻¹ ist.

## Claims

1. A process for preparing phthalic anhydride by gas-phase oxidation of xylene, naphthalene or mixtures thereof in a shell-and-tube reactor thermostatted by means of a heat transfer medium over three or more different annular fixed-bed support catalysts arranged in zones, wherein the process is carried out so that the maximum temperature in the second catalyst zone in the flow direction is from 10 to 40°C lower than the maximum temperature in the first catalyst zone, the maximum temperature in the third zone from the gas inlet is from 30 to 100°C lower than that in the first catalyst zone and the maximum temperature in the first catalyst zone is less than 470°C.

2. The process according to claim 1, wherein the maximum temperature in the third catalyst zone is from 40 to 80°C lower than in the first catalyst zone.

3. The process according to any of the preceding claims, wherein the temperature difference between the maximum temperature in the first catalyst zone and the maximum temperature in the second and third catalyst zones is controlled by means of the bed length ratio of the catalyst zones.

4. The process according to claim 3, wherein the bed length of the first catalyst zone is more than 30% of the length of the total catalyst bed.

5. The process according to claim 3, wherein the bed length of the first catalyst zone is more than 40% of the length of the total catalyst bed.

6. The process according to claim 1, wherein the temperature difference between the maximum temperature in the first catalyst zone and the maximum temperature in the second catalyst zone is controlled via the temperature of the heat transfer medium.

7. The process according to any of the preceding claims, wherein the maximum temperature in the first catalyst zone is less than 450°C.

8. The process according to any of the preceding claims, wherein a gas phase having a loading of from 80 to 140 g of o-xylene and/or naphthalene per standard m³ of gas phase is used.

9. The process according to any of the preceding claims, wherein the temperature of the heat transfer medium is s 360°C.

10. The process according to any of the preceding claims, wherein the space velocity of the gas mixture is ≥ 2000 h⁻¹.

## Revendications

1. Procédé de préparation d'anhydride d'acide phtalique par oxydation en phase gazeuse de xylol, de naphtaline ou de mélanges de ces substances dans un réacteur à faisceau tubulaire thermostatisé à l'aide d'un caloporteur à trois ou plus de trois différents catalyseurs annulaires à lit fixe disposés sous forme de couches, auquel cas le procédé est exécuté de telle façon que la température maximale de la deuxième couche du catalyseur dans le sens de l'écoulement est inférieure de 10 à 40° C à la température maximale relevée dans la première couche du catalyseur, la température maximale de la troisième couche relevée après aspiration des gaz est inférieure de 30 à 100° C à celle de la première couche du catalyseur et la température maximale de la première couche du catalyseur est inférieure à 470° C.

2. Procédé selon la revendication 1, **caractérisé en ce que** la température maximale de la troisième couche du catalyseur est inférieure de 40 à 80° C à la première couche du catalyseur.

3. Procédé selon l'une quelconque des revendications précédentes, **caractérisé en ce que** la différence de température entre la température maximale de la première couche du catalyseur et la température maximale des deuxième et troisième couches du catalyseur est contrôlée via le rapport de la longueur de propagation des couches du catalyseur.

4. Procédé selon la revendication 3, **caractérisé en ce que** la longueur de propagation de la première couche du catalyseur dépasse de 30 % la longueur de l'ensemble du lit catalytique.

5. Procédé selon la revendication 3, **caractérisé en ce que** la longueur de propagation de la première couche du catalyseur dépasse de 40 % celle de l'ensemble du lit catalytique.

6. Procédé selon la revendication 1, **caractérisé en ce que** la différence de température entre la température maximale des première et deuxième couches du catalyseur est contrôlée via la température du caloporteur.

7. Procédé selon l'une quelconque des revendications précédentes, **caractérisé en ce que** la température maximale de la première couche du catalyseur est inférieure à 450° C.

8. Procédé selon l'une quelconque des revendications précédentes, **caractérisé en ce que** l'on recourt à une phase gazeuse avec une charge de 80 à 140 g d'o-xylol et/ou de naphtaline par Nm³.

9. Procédé selon l'une quelconque des revendications précédentes, **caractérisé en ce que** la température du caloporteur est inférieure ou égale à 360° C.

10. Procédé selon l'une quelconque des revendications précédentes, **caractérisé en ce que** la vitesse spatiale du mélange gazeux est supérieure ou égale à 2000 h⁻¹.
